(19) Europäisches Patentamt / European Patent Office / Office européen des brevets

(11) **EP 2 537 969 A1**

(12) **EUROPEAN PATENT APPLICATION**
published in accordance with Art. 153(4) EPC

(43) Date of publication:
**26.12.2012 Bulletin 2012/52**

(51) Int Cl.:
***D04H 1/54*** *(2012.01)*     ***A61F 13/511*** *(2006.01)*

(21) Application number: **11744827.4**

(86) International application number:
**PCT/JP2011/054208**

(22) Date of filing: **18.02.2011**

(87) International publication number:
**WO 2011/102544 (25.08.2011 Gazette 2011/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2010 JP 2010035387**

(71) Applicant: **Unicharm Corporation Ehime 799-0111 (JP)**

(72) Inventors:
• **UEMATSU, Katsuhiro Kanonji-shi Kagawa 769-1602 (JP)**

• **OBA, Toru Kanonji-shi Kagawa 769-1602 (JP)**
• **MIZUTANI, Satoshi Kanonji-shi Kagawa 769-1602 (JP)**

(74) Representative: **Knights, Rupert Saunders & Dolleymore LLP 9 Rickmansworth Road Watford WD18 0JU (GB)**

(54) **NONWOVEN-FABRIC SHEET AND PROCESS FOR PRODUCING SAME**

(57)     Provided are a nonwoven-fabric sheet having a high liquid permeation rate and a process for producing the sheet. The nonwoven-fabric sheet comprises two layers, a first layer (4) and a second layer (5), and is characterized in that the first layer (4) has a plurality of ridges (2) and grooves (3) which extend in parallel in the lengthwise direction, that the first layer comprises heat-fusible fibers and the second layer comprises crimped fibers, and that the sheet has an average fiber angle of 70 or less in a cross-section parallel to the lengthwise direction.

The nonwoven-fabric sheet can be produced by stacking a web comprising fibers having latent crimping properties and a web comprising heat-fusible fibers, ejecting a fluid from a plurality of nozzles arranged in the width direction, while conveying the stack, to form ridges (2) and grooves (3), heating the stack using a means that reduces resistance against the crimping properties of the fibers having latent crimping properties, thereby crimping the fibers having latent crimping properties, and then fusion-bonding the heat-fusible fibers.

Fig.2

**Description**

TECHNICAL FIELD

The present invention relates to a nonwoven fabric sheet having a high liquid permeation rate, and a production method thereof.

BACKGROUND ART

Japanese Unexamined Patent Publication No. 2009-30218 discloses a liquid-pervious nonwoven fabric in which the average fiber angle in the cross-section parallel to the width direction is set to 75° or less so as to reduce the permeation resistance and improve the liquid permeability, and in Examples thereof, a nonwoven fabric having an average fiber angle of 67.4 to 74.7° is described. In the method disclosed in Patent Document 1, a web is fixed on a suction drum rotating in the machine direction MD, and ridges and grooves are formed by air jetting nozzle assemblies disposed at predetermined spacing, whereby the "average fiber angle" in ridges is small.

SUMMARY OF INVENTION

TECHNICAL PROBLEM

The recent speeding-up of the nonwoven fabric production line has resulted in an increase in the line tension and in order to stabilize the delivery step, the nonwoven fabric is always in a state of tension in the machine direction (length direction). Therefore, in the method of fixing a web on a suction drum rotating in the machine direction and forming ridges and grooves by means of air jetting nozzle assemblies disposed at a predetermined, even when the volume of heated and pressurized air blown from the air jetting nozzle is increased to move the fibers sideways in the width direction and thereby decrease the average fiber angle in the cross-section parallel to the width direction, a tensile load due to the line tension places a load in the machine direction after a thermal fusion step, and in a conventional case, since a sheet is formed by melting and fixing fiber intersection points of mainly core-sheath composite fibers (thermoplastic resin) to allow for no presence of a coiled three-dimensional crimp or an elastomer in the fiber network, the stretched sheet lacks the ability to recover from a stretched state and the ridges formed are deformed in the machine direction, resulting in a large average fiber angle in the cross-section parallel to the length direction and a reduction in liquid permeability.

SOLUTION TO PROBLEM

The present inventors have found that when a web comprising a latent crimping fiber is stacked onto a web comprising a heat-fusible fiber and having little resistance against shrinking of the latent crimping fiber in the length direction by crimping, the stack is heat-treated to crimp the latent crimping fiber, and therefore a nonwoven fabric sheet having a small average fiber angle in the cross-section parallel to the length direction can be obtained. The present invention has been accomplished based on this finding.

That is, a first invention is a nonwoven fabric sheet having length, width and thickness directions crossing each other at right angles and having a top surface and a bottom surface in the thickness direction, the top surface being subjected to formation of a plurality of ridges extending parallel in the length direction and a plurality of grooves extending in the length direction between adjacent ridges, wherein

the nonwoven fabric sheet comprises two layers of a first layer on the top surface side and a second layer on the bottom surface side, the first layer comprises a heat-fusible fiber, the second layer comprises a crimped fiber, and

an average fiber angle in the cross-section parallel to the length direction is not more than 70°, wherein acute intersection angles including 90° and obtuse intersection angles larger than 90° are formed by the intersection of a fiber constituting the first layer, which appears in the cross-section parallel to the length direction passing through the apex of the ridge when the nonwoven fabric sheet is placed on a horizontal plane, with a line perpendicular to the horizontal plane, and the average fiber angle in the cross-section parallel to the length direction is an average value of acute intersection angles.

Preferably, an average fiber angle in the cross-section parallel to the width direction is not more than 65°, wherein acute intersection angles including 90° and obtuse intersection angles larger than 90° are formed by the intersection of a fiber constituting the first layer, which appears in the cross-section parallel to the width direction when the nonwoven fabric sheet is placed on a horizontal plane, with a line perpendicular to the horizontal plane passing through the apex of the ridge, and the average fiber angle in the cross-section parallel to the width direction is an average value of acute intersection angles.

Preferably, the crimped fiber is a coiled three-dimensional crimped fiber.

Preferably, the thickness of the ridge is from 0.3 to 5 mm, the difference in height between the apex of the ridge and the bottom of the groove is from 0.1 to 3 mm, the width of the ridge is from 1 to 10 mm, and the width of the groove is from 0.5 to 7 mm.

Preferably, the basis weight of the first layer is from 10 to 100 g/m2 and the basis weight of the second layer is from 5 to 70 g/m2.

Preferably, the crimped fiber is contained in a large proportion in the ridge compared with the groove.

A second invention is a method for producing a nonwoven fabric sheet comprising:

a) a step of passing a fiber aggregate comprising a latent crimping fiber through a carding machine and thereby opening out the fibers to form a web containing a latent crimping fiber,

b) a step of passing a fiber aggregate comprising a heat-fusible fiber through a carding machine and thereby opening out the fibers to form a web comprising a heat-fusible fiber,

c) a step of laminating together the web comprising a latent crimping fiber and the web comprising a heat-fusible fiber to form a stacked web,

d) a step of spraying a fluid on the heat-fusible fiber-comprising web-side surface of the stacked web from a plurality of nozzles arranged in the width direction, while conveying the stacked web, to form a plurality of ridges extending parallel in the length direction and a plurality of grooves extending in the length direction between adjacent ridges on the stacked web,

f) a step of heating the web having formed thereon ridges and grooves, at a temperature lower than the fusion temperature of the heat-fusible fiber but high enough for the latent crimping fiber to develop crimp, by using means for reducing the resistance to crimp development of the latent crimping fiber, and

g) a step of heating the web in which the latent crimping fiber is crimped, at a temperature not lower than the fusion temperature of the heat-fusible fiber and thereby fusion-bonding the heat-fusible fibers to each other at the site of the fibers intersecting one another.

The means for reducing the resistance to crimp development of the latent crimping fiber is preferably a method of conveying the web at a low conveying speed compared with the last step.

The means for reducing the resistance to crimp development of the latent crimping fiber is preferably a floating drier.

The method preferably comprises, between the step d and the step f, e) a step of conveying the web having formed thereon ridges and grooves to the step f.

The method preferably comprises, after the step g, h) a step of cooling the web in which the heat-fusible fibers are fusion-bonded to each other.

A third invention is an absorbent article comprising, as a surface sheet, the nonwoven fabric sheet of the first invention.

ADVANTAGEOUS EFFECTS OF INVENTION

In the nonwoven fabric sheet of the first invention, the average fiber angle in the cross-section parallel to the length direction is 70° or less and therefore, fast liquid permeation is permitted.

In the method for producing a nonwoven fabric sheet of the second invention, a web containing a latent crimping fiber and a web containing a heat-fusible fiber are laminated together and the latent crimping fiber is crimped through a heat treatment by using means for reducing the resistance against crimp development of the latent crimping fiber, so that a nonwoven fabric sheet having a small average fiber angle in the cross-section parallel to the length direction can be obtained.

In the absorbent article of the third invention, a surface sheet permitting fast liquid permeation is used and therefore, a liquid is less likely to accumulate on the surface sheet.

BRIEF DESCRIPTION OF DRAWINGS

Fig. 1 is an enlarged perspective view of the nonwoven fabric sheet of the present invention.

Fig. 2 is an enlarged view of the cross-section in the width direction of the nonwoven fabric sheet of the present invention.

Fig. 3 illustrates one example of the production process of the nonwoven fabric sheet of the present invention.

Fig. 4 illustrates a plurality of nozzles arranged in the width direction, used in the step d.

Fig. 5 illustrates one example of the step d different from Fig. 3.

Fig. 6 illustrates one example of the floating drier which can be used in the step f.
Fig. 7 illustrates a plan view photograph of the nonwoven fabric sheet produced in Example 1.
Fig. 8 illustrates a micrograph of the cross-section of the nonwoven fabric sheet produced in Example 1.
Fig. 9 illustrates a micrograph of the cross-section of the nonwoven fabric sheet in the conventional case.
Fig. 10 is a view for explaining the method to measure the average fiber angle $\theta$.

DESCRIPTION OF EMBODIMENTS

The present invention is described below based on its preferred embodiments by referring to the drawings.

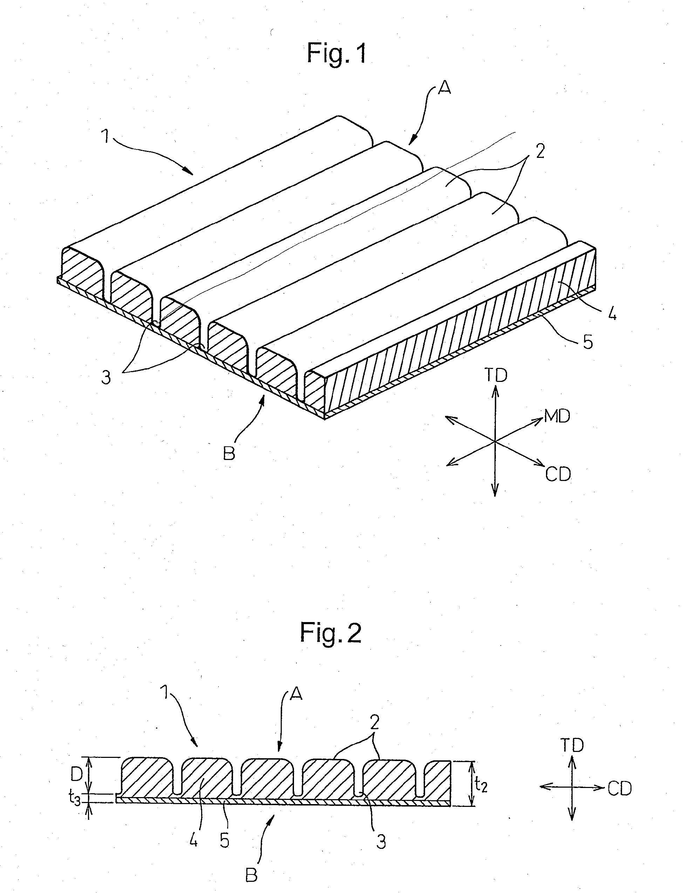

Fig. 1 is an enlarged perspective view of the nonwoven fabric sheet of the present invention. The nonwoven fabric sheet 1 of the present invention has a length direction MD, a width direction CD crossing the length direction MD at right angles, and a thickness direction TD crossing both directions MD and CD at right angles, and the top surface and the bottom surface in the thickness direction TD are denoted by A and B. The length direction MD indicates a machine direction that is the traveling direction of the nonwoven fabric sheet in the step of producing the nonwoven fabric sheet. On the top surface A, a plurality of ridges 2 extending parallel in the length direction and a plurality of grooves 3 extending in the length direction between a ridge 2 and a ridge 2, which are adjacent to each other, are formed.

Fig. 2 is an enlarged view of the cross-section in the width direction CD of the nonwoven fabric sheet 1. As shown in Figs. 1 and 2, the nonwoven fabric sheet 1 has a two-layer structure consisting of a first layer 4 located on the A side and a second layer 5 located on the B side. The first layer 4 and the second layer 5 are integrated on opposing surfaces of two layers by fiber interlacing, heat fusion bonding or other methods.

The second layer 5 contains a crimped fiber. The crimped fiber is a fiber in which the latent crimping fiber used as a raw material of the nonwoven fabric sheet develops crimp by heating when producing the nonwoven fabric sheet. The latent crimping fiber as used herein is a fiber capable of developing crimp when heated, and comprises two or more resins differing in melting point and undergoes three-dimensional crimping due to a change in the heat shrinkage ratio as a result of the difference in the melting point. The resin configuration in the fiber cross-section includes a core-sheath eccentric-type fiber and a side-by-side-type fiber differing in the melting point between right and left components. In the process of producing a nonwoven fabric sheet, when a latent crimping fiber is contained in the second layer and the web is heated to have small resistance to shrinking the latent crimping fiber in the length direction by crimping (for example, by reducing the web conveying speed), the web as a whole can be shrunk in the length direction MD due to crimping of the latent crimping fiber in the second layer and the fiber orientation in the first layer can be directed in the thickness direction TD. The crimped fiber is preferably a coiled three-dimensional crimped fiber. The three-dimensional crimped fiber as used herein is a crimped fiber having a three-dimensional shape like a coiled shape. The coiled crimped fiber is a fiber crimped in a coiled shape. The coiled crimped fiber has a property of returning to its original shape when stretched or shrunk, even after crimping. Since a coiled crimped fiber is contained in the second layer, even when a load in the length direction MD is placed on the nonwoven fabric sheet to stretch the nonwoven fabric sheet, the coiled crimp exerts its force for returning to the original shape and thereby provides stretch recovery, as a result, the first layer shape does not easily deform and the average fiber angle of the first layer is less likely to be changed. By virtue of using a three-dimensional coiled fiber, when heated in the production process, the web as a whole can be shrunk not only in the length direction MD but also in the width direction CD, and the fiber orientation of the first layer can be directed further in the thickness direction TD. Compared with a conventional nonwoven fabric sheet, the nonwoven fabric sheet of the present invention is reduced in the average fiber angle also in the length direction, in addition to the width direction, whereby the resistance encountered by a liquid upon permeating can be more reduced.

The nonwoven fabric sheet of the present invention permits fast liquid permeation, i.e., is excellent in liquid permeability, which is obtained by the fact that the fiber constituting the first layer of the nonwoven fabric sheet of the present invention has a strong propensity to extend in the thickness direction TD without extending in parallel to the top surface A or the bottom surface B. In the present invention, the liquid permeability is evaluated as the later-described artificial urine permeation rate, and the propensity of the first layer 4 to extend in the thickness direction TD is evaluated as an average fiber angle $\theta$.

The average fiber angle $\theta_M$ in the cross-section parallel to the length direction is an average value of acute intersection angles out of acute intersection angles including 90° formed by the intersection of a fiber constituting the first layer and when the nonwoven fabric sheet is placed on a horizontal plane, appearing in the cross-section parallel to the length direction passing through the apex of the ridge with a line perpendicular to the horizontal plane and obtuse intersection angles larger than 90°.

The average fiber angle $\theta_C$ in the cross-section parallel to the width direction is an average value of acute intersection angles out of acute intersection angles including 90° formed by the intersection of a fiber constituting the first layer and when the nonwoven fabric sheet is placed on a horizontal plane, appearing in the cross-section parallel to the width direction with a line perpendicular to the horizontal plane passing through the apex of the ridge and obtuse intersection angles larger than 90°.

In the nonwoven fabric sheet of the present invention, the average fiber angle $\theta_M$ in the cross-section parallel to the length direction is 70° or less. In a conventional case, only the average fiber angle $\theta_C$ in the cross-section parallel to the width direction is small, whereas in the nonwoven fabric sheet of the present invention, the average fiber angle $\theta_M$ in the cross-section parallel to the length direction is also small and therefore, the liquid permeability is enhanced as compared with a conventional case. The average fiber angle $\theta_C$ in the cross-section parallel to the width direction is preferably 65° or less. A coiled three-dimensional crimped fiber is used as the crimped fiber, whereby the average fiber angle $\theta_C$ in the cross-section parallel to the width direction can be made smaller. The fibers in both the length direction MD and the width direction CD are oriented in the thickness direction TD, whereby the liquid permeability is more enhanced as compared with a conventional case.

From the standpoint of improving the absorption and touch of the nonwoven fabric sheet, the thickness $t_2$ (see, Fig. 2) of the ridge 2 is preferably from 0.3 to 5 mm, more preferably from 0.5 to 3 mm. If the thickness exceeds this range, impression on skin from use is increased, whereas if the thickness is less than the range above, even when the average fiber angle $\theta$ of the nonwoven fabric sheet is small, this is surpassed by the effect of the small thickness of the nonwoven fabric as a whole and the liquid permeability is reduced.

From the standpoint of forming a groove 3 suited to keep a liquid even when excreted in a large amount on the nonwoven fabric sheet 1 from widely running on the surface, the difference D in height between the apex of the ridge 2 and the bottom of the groove 3 is preferably from 0.1 to 3 mm, more preferably from 0.3 to 2 mm. If the difference exceeds this range, the height of the ridge 2 inevitably increases and thereby resulting in an uncomfortable feeling, whereas if the difference is less than the range above, the proportion of the ridge 2 in the entire nonwoven fabric sheet 1 decreases and in turn, the proportion of a fiber having a small fiber angle contained in the ridge 2 in the entire nonwoven fabric sheet 1 decreases, as a result, liquid permeability is reduced.

The thickness $t_3$ of the groove 3 is preferably 4.9 mm or less, more preferably 2.7 mm or less. From the standpoint of forming a groove 3 suited to keep a liquid even when excreted in a large amount from widely running on the surface, the thickness $t_3$ of the groove 3 is preferably as small as possible.

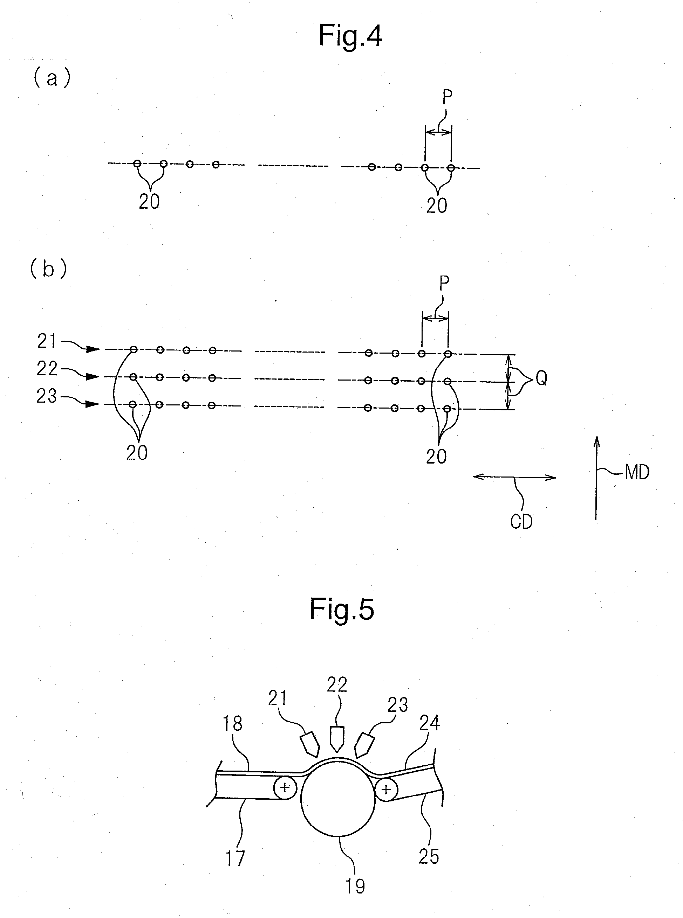

The width of the ridge 2 in the width direction CD of the nonwoven fabric sheet 1 is, in view of touch to skin, preferably from 1 to 10 mm, more preferably from 2 to 5 mm. From the same standpoint, the width of the groove 3 in the width direction CD of the nonwoven fabric sheet 1 is preferably from 0.5 to 7 mm, more preferably from 0.7 to 3 mm. The ridge 2 and the groove 3 may be formed to have the same width or may be formed to differ in the width. The widths of the ridge 2 and the groove 3 may be variously changed according to the pitch (interval between adjacent nozzles) and aperture of a fluid spraying nozzle used in the later-described production method. For example, the width of the ridge 2 can be widened by increasing the pitch P (see, Fig. 4) of the nozzle, and the width of the groove 3 can be widened by increasing the aperture of the nozzle. Also, the height of the ridge 2 can be decreased by narrowing the pitch of the fluid spraying nozzle, and conversely, the height of the ridge 2 can be increased by widening the pitch of the fluid spraying nozzle. Furthermore, when fluid spraying nozzles are arranged to have alternately a narrow pitch and a wide pitch, ridges 2 differing in height can be alternately formed. In addition, when the height of the ridge 2 is thus partially changed, the contact area with skin is decreased, and the load on skin can be advantageously reduced.

The first layer 4 is composed of a fiber layer containing a heat-fusible fiber as an essential fiber. In the first layer 4, the heat-fusible fiber is contained preferably in a proportion of 30 to 100 percent by weight. The heat-fusible fiber is a fiber enabling heat fusion bonding of intersection points of fibers to each other. In the first layer 4, the heat-fusible fibers intersecting one another are joined at the site of the fibers intersecting one another resulting from melting of the thermoplastic resin forming the fiber. Heat-fusible fibers are mixed in the first layer 4 and joined at the site of the fibers intersecting one another, whereby the shape retentivity of the convex three-dimensional shape of the ridge 2 is enhanced and the average fiber angle $\theta$ can be prevented from increasing due to friction or pressure during use.

From the standpoint of improving skin touch and absorbency of the nonwoven fabric sheet, the fineness of the fiber used in the first layer 4 is preferably from 1 to 8 dtex, more preferably from 1.8 to 3.3 dtex. If the fineness exceeds this range, the user feels discomfort due to thickness of the fiber, whereas if the fineness is less than the range above, the distance between fibers becomes too short and a liquid readily encounters resistance, leading to reduction in the permeation rate. Also, in view of suitability for carding, the fiber length used is from 15 to 100 mm, preferably from 38 to 51 mm.

The first layer 4 may be composed of one kind of heat-fusible fiber or two or more kinds of heat-fusible fibers or may be configured to contain a non-heat-fusible fiber in addition to the heat-fusible fiber. For example, one or more fibers may be arbitrarily selected from a regenerated fiber such as rayon, a semisynthetic fiber such as acetate, a natural fiber such as cotton and wool, and a synthetic fiber such as polypropylene, polyethylene, polyester, polyamide, polyvinyl chloride and vinylon, and used. The fiber cross-sectional shape and the like thereof are not limited and, for example, a split type composite fiber or a modified cross-section fiber may be arbitrarily used. In the case of containing a non-heat-fusible fiber, the amount of the heat-fusible fiber is preferably from 30 to 95 percent by weight, more preferably from 70 to 90 percent by weight, based on the weight of the first layer 4.

As the heat-fusible fiber contained in the first layer 4, a fiber composed of a thermoplastic polymer material is suitably used. Examples of the thermoplastic polymer material include a polyolefin such as polyethylene and polypropylene, polyester such as polyethylene terephthalate, and a polyamide. A core-sheath type or side-by-side type composite fiber composed of a combination of these thermoplastic polymer materials may be also used.

The second layer 5 is composed of a fiber layer containing a crimped fiber whose crimp is developed by heating in the production process. In the second layer 5, the crimped fiber is preferably contained in a proportion of 30 to 100 percent by weight. Examples of the latent crimping fiber forming the second layer 5 include a core-sheath eccentric composite fiber and a side-by-side composite fiber each using, as the component, two kinds of thermoplastic polymer materials differing in the shrinkage ratio. From the standpoint of shrinking the second layer 5 to orient the fibers constituting the first layer 4 in the thickness direction TD and thereby enhance the liquid permeability, a combination giving a web with a shrinkage ratio of at least 40% is preferred. Examples thereof include a combination of a polyolefin polypropylene copolymer and a polypropylene. Also, from the standpoint of improving the liquid permeability, the fineness used is preferably from 1 to 11 dtex, more preferably from 2.2 to 5.6 dtex. Furthermore, in view of suitability for carding, the fiber length used is from 15 to 100 mm, preferably from 38 to 51 mm.

The second layer 5 may be composed of one kind of crimped fiber or two or more kinds of crimped fiber or may be configured to contain, in addition to the crimped fiber, a non-crimped fiber or another fiber incapable of undergoing heat fusion bonding with the fiber. For example, one or more fibers may be arbitrarily selected from a regenerated fiber such as rayon, a semisynthetic fiber such as acetate, a natural fiber such as cotton and wool, and a synthetic fiber such as polypropylene, polyethylene, polyester, polyamide, polyvinyl chloride and vinylon, and used. The fiber cross-sectional shape and the like thereof are not limited and, for example, a split-type composite fiber or a modified cross-section fiber may be used. In this case, from the standpoint of developing sufficient heat shrinkage, the crimped fiber is preferably used in a proportion of 30 percent by weight or more, more preferably 80 percent by weight or more.

The crimped fiber is preferably contained in a large proportion in the ridge as compared with the groove. That is, the absolute amount of crimped fiber (the basis weight of the second layer) is preferably larger in the ridge than in the groove. In using the nonwoven fabric of the present invention as the surface sheet of a disposable diaper or a sanitary napkin, when deformation of the product occurs due to twisting or the like during use, by virtue of containing the crimped fiber in a large proportion in the ridge as compared with the groove, the coiled crimp readily exerts its force for returning to the original shape and thereby provides stretch recovery, as a result, the first layer shape is hardly deformed and the average fiber angle of the first layer in the ridge is less likely to be changed.

At the time of spraying a fluid on the stacked web from a nozzle in the step d, fibers beneath the nozzle are shifted parallel in the width direction CD and accumulated between adjacent nozzles and in turn, the basis weight of the latent crimping fiber in the ridge can be increased, so that when the latent crimping fiber develops crimp and becomes a crimped fiber, a sheet where the crimped fiber is contained in a large proportion in the ridge as compared with the groove can be produced.

The temperature at which the latent crimping fiber used in the second layer 5 starts shrinking must be lower than the temperature allowing for heat fusion bonding of the mutual intersection point of fibers used in the first layer 4. By shrinking the second layer 5 before fixing the shape by heat fusion bonding of the fibers of the first layer 4, the fibers constituting the first layer 4 can be oriented in the thickness direction TD.

In view of touching skin when incorporated into a product, the basis weight of the nonwoven fabric sheet after the shrinking step is preferably from 15 to 150 $g/m^2$, more preferably from 25 to 80 $g/m^2$. For example, the basis weight of the first layer 4 after the shrinking step is preferably from 10 to 100 $g/m^2$, more preferably from 15 to 60 $g/m^2$, and the basis weight of the second layer 5 after the shrinking step is preferably from 5 to 70 $g/m^2$, more preferably from 10 to 50 $g/m^2$. If the basis weight of the first layer 4 is too small, due to a small number of fibers, the layer is crushed by a compressive force such as body pressure of the wearer and the fibers are directed in the planar direction, as a result, the liquid permeability is deteriorated. Conversely, if the basis weight is too large, even when the fibers are directed in the thickness direction TD, since the fiber amount is increased, the resistance encountered by a liquid on permeating becomes large and this is not preferred. If the basis weight of the second layer 5 is too small, the fibers of the first layer 4 cannot be gathered in either the length direction MD or the width direction CD and cannot be directed in the thickness direction TD. Conversely, if the basis weight is too large, the shrinking action occurs excessively to cause irregular deformation of the surface portion of the ridge and this disadvantageously provides a feeling of strangeness to the wearer.

The nonwoven fabric sheet is preferably hydrophilized. Examples of the method for hydrophilization include a method where a fiber treated with a hydrophilizing agent is used as a raw material, and a method where a fiber having kneaded therein a hydrophilizing agent is used as a raw material. Hydrophilization may be also performed by producing a nonwoven fabric sheet and then coating a surfactant thereon.

The method for producing a nonwoven fabric sheet of the present invention is described below.

One embodiment of the method for producing a nonwoven fabric sheet of the present invention includes the following steps, provided that out of the following steps, the step e and the step h are not essential:

a) a step of passing a fiber aggregate comprising a latent crimping fiber through a carding machine and thereby opening out the fibers to form a web comprising a latent crimping fiber,

b) a step of passing a fiber aggregate comprising a heat-fusible fiber through a carding machine and thereby opening out the fibers to form a web comprising a heat-fusible fiber,

c) a step of laminating together the web comprising a latent crimping fiber and the web comprising a heat-fusible fiber to form a stacked web,

d) a step of spraying a fluid on the heat-fusible fiber-comprising web-side surface of the stacked web from a plurality of nozzles arranged in the width direction, while conveying the stacked web, to form a plurality of ridges extending parallel in the length direction and a plurality of grooves extending in the length direction between adjacent ridges on the stacked web,

e) a step of conveying the web having formed thereon ridges and grooves to the step f,

f) a step of heating the web having formed thereon ridges and grooves, at a temperature lower than the fusion temperature of the heat-fusible fiber but high enough for the latent crimping fiber to develop crimp, by using means for reducing the resistance to crimp development of the latent crimping fiber,

g) a step of heating the web in which the latent crimping fiber is crimped, at a temperature not lower than the fusion temperature of the heat-fusible fiber and thereby fusion-bonding the heat-fusible fibers to each other at the site of the fibers intersecting one another, and

h) a step of cooling the web in which the heat-fusible fibers are fusion-bonded to each other.

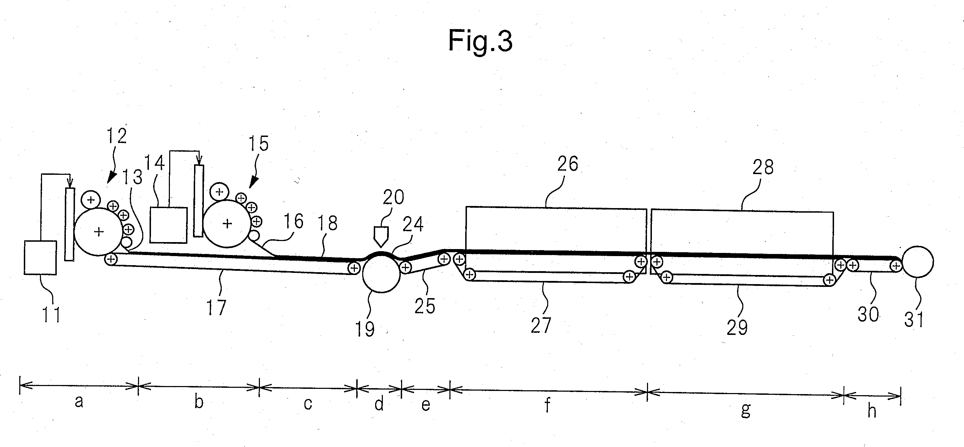

Fig. 3 illustrates one example of the production process of the nonwoven fabric sheet of the present invention, but the present invention is not limited to this example.

In Fig. 3, a indicates step a, b indicates step b, c indicates step c, d indicates step d, e indicates step e, f indicates step f, g indicates step g, and h indicates step h.

In the step a, a fiber aggregate comprising a latent crimping fiber is conveyed to a carding machine 12 from a vessel 11 and passed through the carding machine 12, thereby opening out the fibers, to form a web 13 comprising a latent crimping fiber. The formed web 13 comprising a latent crimping fiber is conveyed on an endless belt 17.

In the step b, a fiber aggregate comprising a heat-fusible fiber is conveyed to a carding machine 15 from a container 14 and passed through a carding machine 15, thereby opening out the fibers, to form a web 16 comprising a heat-fusible fiber. The order of the step a and the step b is not limited.

In the step c, the web 13 comprising a latent crimping fiber and the web 16 comprising a heat-fusible fiber are laminated together to form a stacked web 18. In Fig. 3, an embodiment where the formed web 16 comprising a heat-fusible fiber is stacked on the web 13 comprising a latent crimping fiber on the endless belt 17 is illustrated, but it is not necessarily required to stack the web 16 comprising a heat-fusible fiber on the web 13 comprising a latent crimping fiber, and the stacked web 18 may be formed by stacking the web 13 comprising a latent crimping fiber on the web 16 comprising a heat-fusible fiber. That is, either the step a or the step b may come first, and when conveying the stacked web, the web 16 comprising a heat-fusible fiber may be present above or the web 13 comprising a latent crimping fiber may be present above.

In the step d, the stacked web 18 rides on a suction drum 19 rotating in the length direction MD, and a fluid is sprayed on the heat-fusible fiber comprising web side surface of the stacked web from a plurality of nozzles 20 arranged in the width direction. The nozzle 20 can spray a fluid toward the peripheral surface of the suction drum 19 and is spaced a predetermined distance from the peripheral surface of the suction drum 19. As for the plurality of nozzles 20 arranged in the width direction, a plurality of nozzles 20 are attached to a pipe (not shown) extending in the axial direction of the suction drum 19, i.e., in the width direction CD, with a predetermined spacing P (see, Fig. 4(a)).

The fluid spraying nozzles 20 may be configured by arranging one line of nozzles but in view of pushing aside of the fibers, preferably configured by arranging two or more lines of nozzles. For example, as shown in Fig. 4(b), in a case where nozzle lines 21, 22 and 23 are arranged in a preferred attachment state, nozzles 20 in each of the nozzle lines 21, 22 and 23 are adjusted to be located on the same line in the length direction MD. Also, the nozzle lines 21, 22 and 23 may be disposed, as shown in Fig. 5, at intervals of 30° in the circumferential direction of the suction drum 19, and the nozzles 20 in each of the nozzle lines 21, 22 and 23 can be attached to a pipe, for example, such that the pitch P in the width direction CD is 5 mm. From the nozzle lines 21, 22 and 23, a fluid at a predetermined temperature can be sprayed into a predetermined air volume. The fluid sprayed from a plurality of nozzles 20 are adjusted not to disorder the distributed state of heat-fusible fibers in the first layer 4, by the fluid itself or by interference between fluids from respective nozzles 20. On this account, for example, in the case where a stacked web 18 having a total basis weight of 35 $g/m^2$ passes the peripheral surface of a suction drum 19 with a diameter of 500 mm in 0.5 seconds and nozzles 20 of each of the nozzle lines 21, 22 and 23 are disposed at a pitch P of 5 mm in the width direction CD and where the spacing dimension from the peripheral surface of the suction drum 19 is adjusted to from 5 to 8 mm, the stacked web is preferably conditioned to have a thickness of approximately from 2 to 5 mm by suction of the suction drum 19 and then passed below the nozzles 20. At this time, it is preferred that the aperture of the nozzle 20 used is approximately from 0.5 to 1.5 mm, the spraying speed of fluid from the nozzle 20 is from 50 to 700 m/sec, and the suction force of the suction drum 19 is from 2 to 7 m/sec in terms of wind velocity.

The stacked web on the peripheral surface of the suction drum 19 passes below the nozzle lines 21, 22 and 23. While spraying a fluid toward the stacked web from the nozzle lines 21, 22 and 23, the suction drum 19 executes suction for suctioning the fluid.

In the stacked web sprayed with the fluid, fibers beneath respective nozzles 20 in each of the nozzle lines 21, 22 and 23 are shifted parallel in the width direction CD and accumulated between a nozzle 20 and a nozzle 20 adjacent thereto to form a ridge 2. On the other hand, a groove 3 is formed beneath each nozzle 20.

Examples of the fluid sprayed from the nozzle 20 include a gas adjusted to an ordinary temperature or a predetermined temperature, and an aerosol containing fine solid or liquid particles in the gas. Examples of the gas include air and nitrogen. The gas may be liquid vapor. The aerosol is a fluid formed by dispersing a liquid or a solid in a gas, and examples thereof are described below. Examples include those obtained by dispersing an ink for coloring, a softener such as silicone for increasing the flexibility, a hydrophilic or water-repellent activator for controlling antistatic property or wettability, an inorganic filler such as titanium oxide and barium sulfate for increasing the fluid energy, a powder bond such as polyethylene for increasing the fluid energy and at the same time, enhancing the property of maintaining an irregular formation during heat treatment, an antihistamine such as diphenhydramine hydrochloride and isopropylmethylphenol for preventing itching, a humectant, or a bactericide. The solid as used herein encompasses a gel-like solid.

The fluid sprayed from the nozzle 20 is preferably a pressurized, heated and compressed air. By blowing a pressurized, heated and compressed air from the nozzle 20, fibers beneath the nozzle are parallelly shifted in the width direction CD and accumulated between a nozzle 20 and a nozzle 20 adjacent thereto to form a temporary ridge. The fibers of the first layer are parallelly shifted in the width direction CD to thereby direct the fiber orientation in the thickness direction. At this time, the heat-fusible fiber of the first layer beneath the nozzle 20 is melted and heat-fusible fibers are fixed to each other, thereby forming a temporary groove. Also, the latent crimping fiber in the second layer, on which the hot air of the nozzle 20 is not directly blown, remains with a latent crimping property.

The step e is a step of conveying the web 24 having formed thereon ridges and grooves in the step d to the step f. In the step e, the web 24 having formed thereon ridges and grooves is conveyed on an endless belt 25. The conveying speed in the step e is the same speed as the conveying speed in the step d or a speed slightly higher than the conveying sped in the step d. The step e may not be necessary, and the web 24 having formed thereon ridges and grooves in the step d may be conveyed from the step d to the step f. However, for stably conveying the web 24, it is preferred to provide the step e.

The step f is a first heat treatment step for developing crimp of the fiber constituting the second layer. The web having formed thereon ridges and grooves in the step d is conveyed to the first heat treatment step f though the conveying step e. In the first heat treatment step f, a first heat treatment drier 26 is provided, and the web 24 on an endless belt 27 is passed through the first heat treatment drier 26 and heat-treated therein. In the first heat treatment step f, the latent crimping fiber in the second layer develops crimp, and the crimp development of the latent crimping fiber in the second layer is effected by performing the heat treatment under the temperature condition not allowing heat-fusible fibers in the first layer to be fusion-bonded and fixed to each other. At the time of the web as a whole being shrunk in both the width direction CD and the length direction MD due to crimp development of the latent crimping fiber in the second layer, since the heat-fusible fibers in the first layer are not fusion-bonded to each other, the orientation of fibers constituting the first layer can be more directed in the thickness direction TD than the orientation in the step d, without preventing crimp development of the latent crimping fiber in the second layer.

Also, at this time, for further directing the orientation in the length direction MD of the first layer to the thickness direction TD, the second layer need to be aggressively shrunk in the length direction MD by the crimp development of the latent crimping fiber in the second layer. In the first heat treatment drier 26, when a tension in the thickness direction MD is placed on the web, shrinkage of the second layer due to crimp development of the latent crimping fiber is likely to occur only in the width direction CD where the degree of freedom is relatively high. Therefore, in the first heat treatment step f, means for reducing resistance to crimp development of the latent crimping fiber is used. In other words, the heat treatment is performed in a state of having small resistance to shrinking of the latent crimping fiber in the length direction by crimping. Examples of the means for reducing the resistance to crimp development of the latent crimping fiber include a method of controlling the conveying speed in the first heat treatment step f to be low compared with the conveying speed in the last step, and a method of using a floating drier.

The last step in the method of controlling the conveying speed in the first heat treatment step f to be low as compared with the conveying speed in the last step is the step e in the case of providing a step e and the step d in the case of not providing a step e. Due to conveyance to the heat treatment drier at a low conveying speed as compared with the last step, sufficient shrinking in the length direction MD due to crimp development of the latent crimping fiber in the second layer can be obtained, and a nonwoven fabric sheet having a small fiber angle $\theta$ in both the cross-section parallel to the length direction and the cross-section parallel in the width direction can be obtained. For example, it is preferred to develop sufficient shrinking in both the width direction CD and the length direction MD and reduce the conveying speed in the first heat treatment step f to be from 90 to 99% of the conveying speed in the last step, where the web is kept from bending and folding.

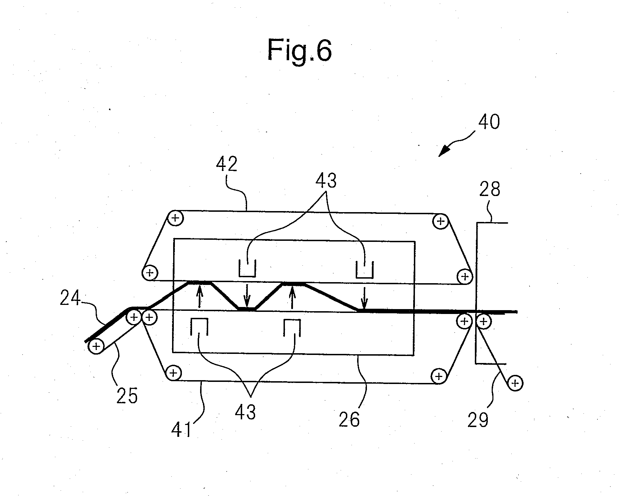

One of the resistances against shrinking by crimp development of the latent crimping fiber is a line tension during conveyance. Another is a friction with the conveyer. In order to reduce the friction, a floating drier 40 shown in Fig. 6 may be utilized. In the floating drier 40, mesh conveyers 41 and 42 are disposed up and down with spacing, a plurality of hot air blowing ports 43 are provided on the inner side (the side opposite the web 24) of each of the mesh conveyers 41 and 42, and hot air is blown toward the other mesh conveyer 42 or 41 while moving the web 24 to the other mesh conveyer 42 or 41, whereby the heat treatment is performed. As shown in Fig. 6, hot air is blown alternately from above and from below in such a manner as blowing hot air from the lower-side mesh conveyor 41 and then blowing hot air from the upper-side mesh conveyor 42, as a result, the web 24 moves up and down and comes to have a portion not contacting with the mesh conveyer 41 or 42, enabling reduction of the resistance against shrinkage due to crimp development of the latent crimping fiber, and this is a preferred method for shrinking the web in both the length direction MD and the width direction CD. The floating drier can be used in combination with the method of controlling the conveying speed in the first heat treatment step f to be low as compared with the conveying speed in the last step and is preferably used in combination.

The step g is a second heat treatment step for fusion-bonding the heat-fusible fibers in the first layer to each other at the site of the fibers intersecting one another. In the second heat treatment step g, a second heat treatment drier 28 is provided, and the web in which the latent crimping fiber is crimped in the step f is loaded on an endless belt 29, passed through the second heat treatment drier 28 and heat-treated therein. In the second heat treatment step g, a heat treatment is performed at a temperature not lower than the fusion temperature of the heat-fusible fiber in the first layer to fusion-bond the heat-fusible fibers to each other at the site of the fibers intersecting one another, whereby the first layer directed in the thickness direction TD in the steps d to f is fixed.

The step h is a step of cooling the web where heat-fusible fibers are fusion-bonded to each other in the step g. The web exited from the second heat treatment drier 28 of the step g is allowed to cool at room temperature while being conveyed on an endless belt 30. By the cooling, fusion-bonding of heat-fusible fibers in the first layer to each other at the site of the fibers intersecting one another is fixed, and the first layer directed in the thickness direction TD in the steps d and f is fixed. The cooled web is taken up by a roll 31.

In the conventional "method of fixing a web on a suction drum rotating in the length direction and forming ridges and grooves by fluid spraying nozzles disposed with a spacing of predetermined dimension", when only the volume of heated and compressed air blown from the fluid spraying nozzle is relied on so as to increase the average fiber angle, the heated and compressed air that cannot be suctioned in the suction treatment produces an excessive reflection flow from the support surface to disorder the fibers, as a result, the touch to skin is impaired. On the other hand, in the present invention, the nonwoven fabric sheet is composed of two layers of first layer and second layer, and the second layer is composed of a latent crimping fiber, making it possible that crimp of the latent crimping fiber is developed in the heat treatment step to push aside the fibers in each of the length direction and the width direction and decrease the average fiber angle $\theta$, the resistance encountered by a liquid upon permeation is reduced by the decrease in the average fiber angle even without unnecessarily increasing the amount of the heated and compressed air blown, and a nonwoven fabric sheet with good touch to skin, in which the fibers on the surface are not disordered, is obtained.

Also, even in the high-speed production, a nonwoven fabric sheet with a small average fiber angle $\theta$ can be stably obtained by employing the production method of the present invention.

A third invention is an absorbent article containing, as a surface sheet, the nonwoven fabric sheet of the first invention. Examples of the absorbent article include a disposable diaper and a sanitary napkin. The absorbent article of the present invention uses a surface sheet permitting fast liquid permeation and therefore, scarcely allows accumulation of a liquid on the surface sheet. Accordingly, when a liquid is excreted during use, the liquid is less likely to accumulate between the skin and the absorbent article and hardly provides a discomfort feeling to the user. In addition, both high liquid permeability and good touch to skin can be satisfied.

EXAMPLES

Example 1

A nonwoven fabric sheet was produced using the production apparatus shown in Fig. 3.

In the step a, a polypropylene/polyolefin polypropylene copolymer latent crimping side-by-side composite fiber (2.6 dtex, fiber length: 51 mm, weight ratio = 50/50, area shrinkage ratio: 80%) was used as the fiber for the second layer (the method for measuring the area shrinkage ratio is described in Evaluation Method later).

In the step b, a polyester/polyethylene core-sheath composite fiber (heat-fusible fiber, 2.6 dtex, fiber length: 51 mm, core/sheath weight ratio = 50/50) was used as the fiber for the first layer.

In the step c, the basis weight of the first layer web was adjusted to 20 g/m$^2$, and the basis weight of the second layer web was adjusted to 15 g/m$^2$.

In the step d, one line of nozzles with an aperture of 1.0 mm and a pitch of 4 mm were used, and air at about 125°C was used as the fluid sprayed and was sprayed at a flow rate of 8.7 L/min per one nozzle. The spacing dimension between the nozzle and the suction drum was set to 5.0 mm, and the suction force of the suction drum was set to 5 m/sec in terms of wind velocity.

In the step e, the conveying speed was set to 10 m/min.

In the step f, the temperature of the first heat treatment drier 26 was set to about 120°C, the wind velocity was set to 0.7 m/s, and the residence time was set to 10 seconds. At this time, the conveying speed was set to 9.0 m/min so that sufficient shrinkage can be developed in both the width direction CD and the length direction MD and bending and folding can be prevented.

In the step g, the temperature of the second heat treatment drier 28 was set to about 138°C, the wind velocity was set to 0.7 m/s, and the residence time was set to 10 seconds.

In the step h, the web was allowed to cool at room temperature.

Through these steps, a nonwoven fabric sheet where the thickness was 1.80 mm, the basis weight was 43 g/m$^2$, the difference in height between a ridge 2 and a groove 3 was 1.4 mm, the width of the ridge 2 was 2.5 mm, the width of the groove 3 was 1.1 mm, the average fiber angle in the cross-section parallel to the length direction was 66.5°, and the average fiber angle in the cross-section parallel to the width direction was 55.1°, was obtained.

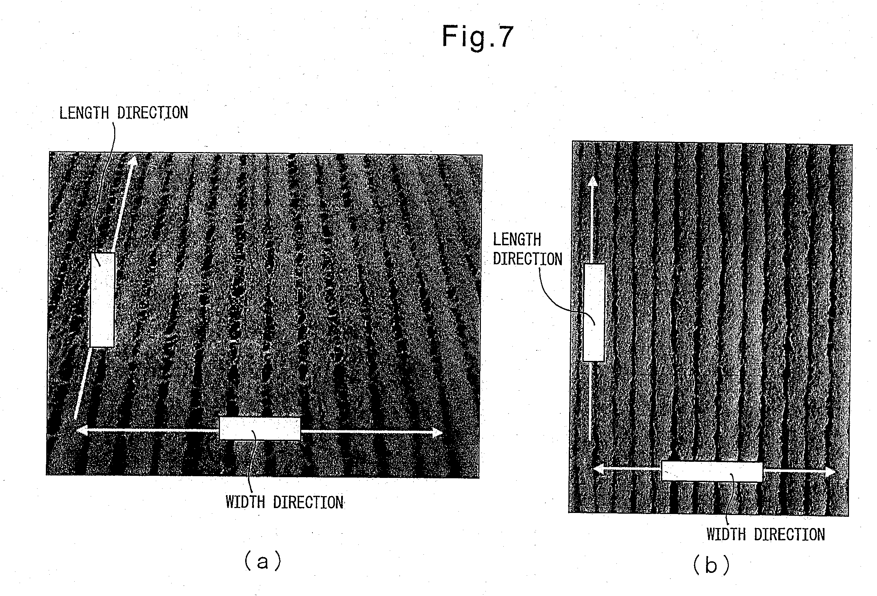

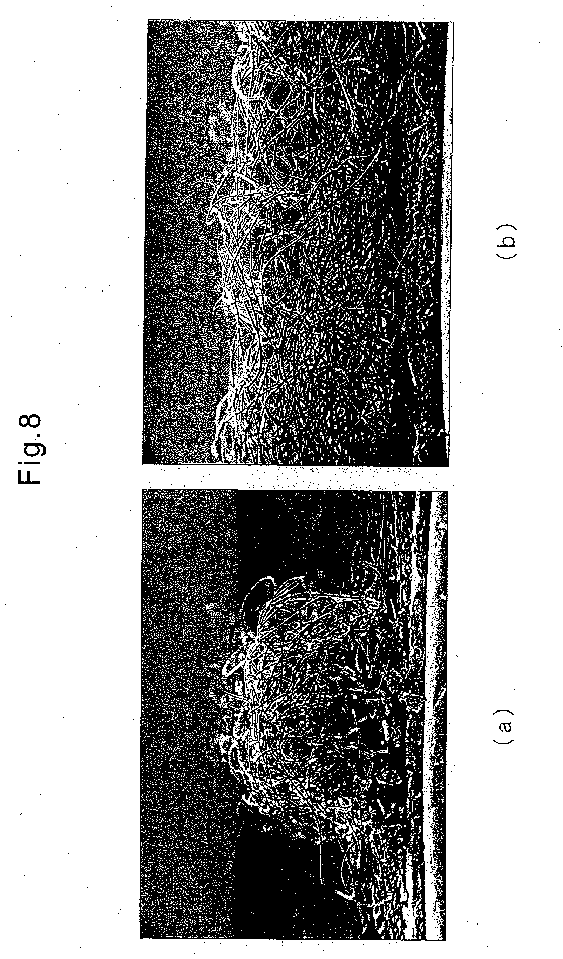

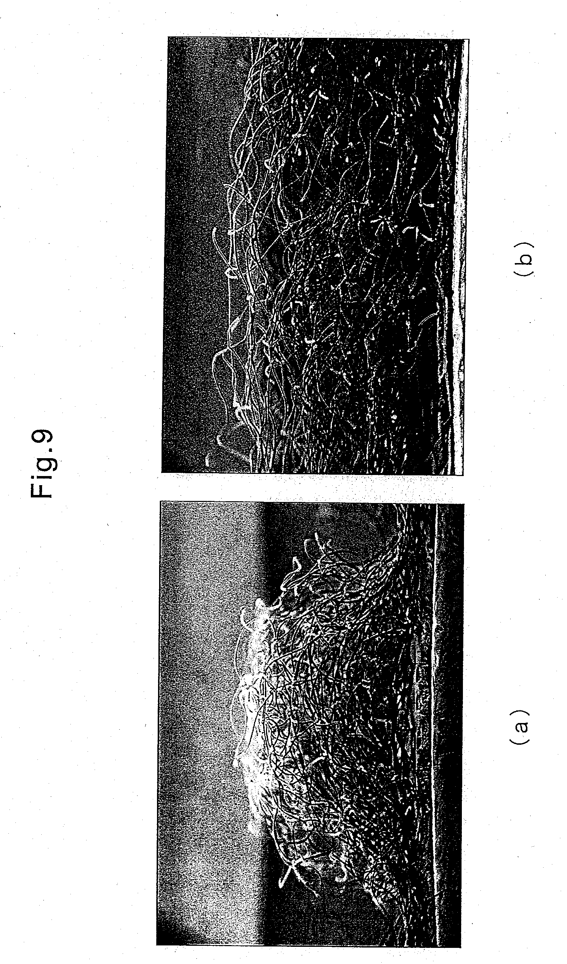

Fig. 7 illustrates a plan view photograph (digital camera) of the nonwoven fabric sheet obtained. Fig. 7(a) is an oblique plan image, and Fig. 7(b) is a plan image. Fig. 8 illustrates a micrograph of the cross-section of the nonwoven fabric sheet obtained. Fig. 8(a) is a micrograph (magnification: 30 times) of the cross-section parallel to the width direction, and Fig. 8(b) is a micrograph (magnification: 30 times) of the cross-section parallel to the length direction passing through the apex of the ridge. For comparison, Fig. 9 shows a micrograph of the cross-section of a nonwoven fabric sheet produced by the method described in Patent Document 1 (hereinafter, sometimes referred to as "conventional nonwoven fabric sheet"). Fig. 9(a) is a micrograph (magnification: 30 times) of the cross-section parallel to the width direction of the conventional nonwoven fabric sheet, and Fig. 9(b) is a micrograph (magnification: 30 times) of the cross-section parallel to the length direction passing through the apex of the ridge of the conventional nonwoven fabric sheet.

Example 2

A nonwoven fabric sheet was produced in the same manner as in Example 1 except that the conveying speed in the step f was changed to 9.5 m/min (that is, the ratio of the conveying speed in the step f to the conveying speed in the step e was changed to 0.95).

Comparative Example 1

A nonwoven fabric sheet was produced in the same manner as in Example 1 except that the conveying speed in the step f was changed to 10 m/min (that is, the ratio of the conveying speed in the step f to the conveying speed in the step e was changed to 1.00).

Comparative Example 2

A nonwoven fabric sheet was produced in the same manner as in Example 1 except that the same polyester/ polyethylene core-sheath composite fiber as the fiber for the first layer was used as the fiber for the second layer. In Comparative Example 2, since the sheet was composed of only a heat-fusible fiber (that is, a latent crimping fiber was not contained), the shrinking force of the web in the length direction MD was not obtained even by reducing the ratio of the conveying speed in the step f to the conveying speed in the step e and therefore, the ratio of the conveying speed in the step f to the conveying speed in the step e was set to 100%.

The nonwoven fabric sheets of Examples 1 and 2 and Comparative Examples 1 and 2 were measured for basis weight, thickness, average fiber angles $\theta_M$ and $\theta_C$, and artificial urine permeation rate. The results are shown in Table 1. Evaluation methods for respective evaluation items are as follows.

[Basis Weight]

The nonwoven fabric sheet is cut into a size of 100 mm x 100 mm, and the weight is measured by an electron balance and converted into the weight per 1 m$^2$. An average of N=10 is determined.

[Thickness]

A thickness measuring device (PEACOCK, measuring surface: $\phi$44 mm, measuring pressure: 3 g/cm$^2$) is used. The sample in an appropriate size (larger than the gauge head) is placed between the measuring table and the gauge head, and after dropping the gauge head from a fixed height, the thickness is measured. An average of N=10 is determined.

[Average Fiber Angle θ]

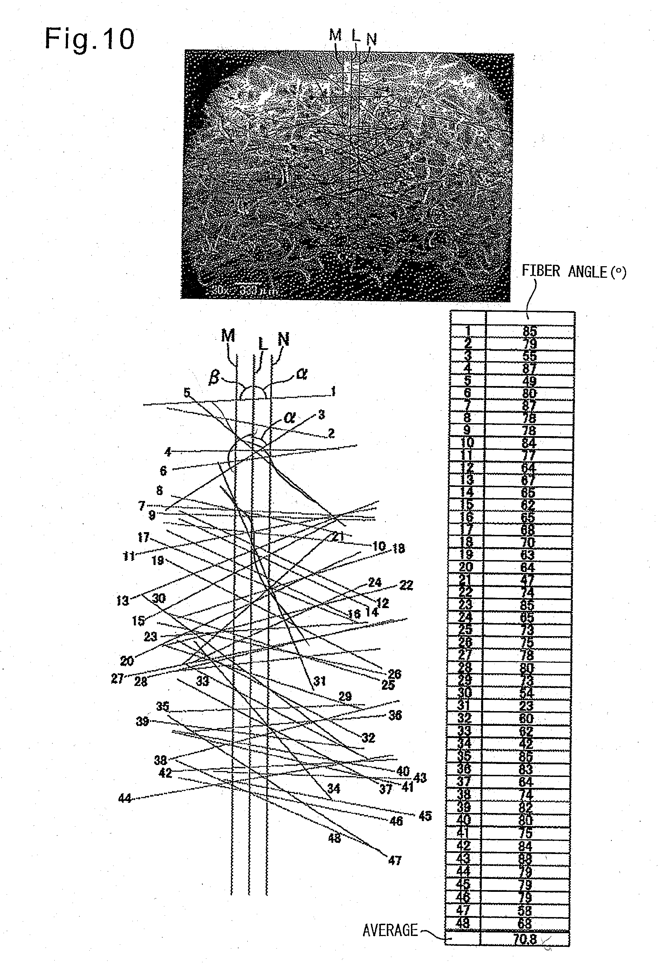

(1) The nonwoven fabric sheet as the sample for measurement is heated at 70°C for 30 minutes to eliminate the folding habit produced in the process of handling the nonwoven fabric sheet and flatten the sample.
(2) The sample is cut to make a cut surface by using a standard replacement blade HA-100B for Kokuyo Cutter Knife HA-7NB (trade name), and the sample is placed on a horizontal plane. At this time, in the case of measuring the average fiber angle $\theta_M$ in the cross-section parallel to the length direction, the sample is cut by a vertical plane parallel to the length direction MD passing through the apex of the ridge to make a cut surface for observation running in parallel with the length direction MD, and in the case of measuring the average fiber angle $\theta_C$ in the cross-section parallel to the width direction, the sample is cut by a vertical plane parallel to the width direction CD to make a cut surface for observation running in parallel with the width direction CD.
(3) The cut surface is observed by an electronic microscope (REAL-SURFACE-VIEW microscope VE-7800 manufactured by Keyence Corporation), and a 30 times magnified photograph of the cutting surface is taken. At the photographing, the region from the top surface to the bottom surface of the sample is brought into the viewing field.
(4) As shown in Fig. 10, in the photograph of the cutting surface, a vertical line L with respect to the horizontal plane is drawn and auxiliary lines M and N are drawn on both right and left sides of the vertical line L with a parallel spacing of 100 $\mu$m from the vertical line L. At this time, in the case of measuring the average fiber angle $\theta_M$ in the cross-section parallel to the length direction, the vertical line L may be drawn at an arbitrary position, but in the case of measuring the average fiber angle $\theta_C$ in the cross-section parallel to the width direction, the vertical line is drawn to pass through the apex of the ridge.
(5) With respect to one fiber intersecting two auxiliary lines M and N, the intersection positions with respective auxiliary lines M and N are marked.
(6) The right and left marks are connected by a straight line, and intersection angles $\alpha$ and $\beta$ between the straight line and the vertical line L on each of two sides of the vertical line L are determined. Out of the determined intersection angles $\alpha$ and $\beta$, the angle of smaller value is taken as the fiber angle.
(7) The fiber angle is determined for all the fibers sufficiently focused on the photograph of the cutting surface to serve as the object of measurement, and an arithmetic average value of the measured fiber angles is defined as "average fiber angle θ". The fibers sufficiently focused on the photograph of the cutting surface are a fiber "appearing in the cross-section" according to the present invention.

[Artificial Urine Permeation Rate]

A tester manufactured by Lenzing Technik is used. The absorption rate (sec) of the nonwoven fabric sheet with a liquid measure of 10 mL is measured by using an artificial urine (72 mN/m, 20°C) as the test solution in accordance with EDANA-ERT §150.3 Liquid Strike Through Time Method.
The artificial urine is prepared by blending 200 g of urea, 80 g of sodium chloride, 8 g of magnesium sulfate, 3 g of calcium chloride and about 1 g of dye (Blue No. 1) to 10 L of ion-exchanged water.

[Area Shrinkage Ratio of Fiber Web]

(1) A web of 200 g/m$^2$ is formed of 100% fiber to be measured.
(2) The web is cut into a predetermined length and a predetermined width, and the area is measured. At this time, in order to reduce the measurement error, the web is preferably cut into a size of approximately 250 mm x 250 mm. The measure area before shrinkage is designated as a.
(3) The sample is left standing in an oven adjusted to 145°C for five minutes.
(4) The length and width after shrinkage are measured, and the area is calculated. The measured area after shrinkage is designated as b.
(5) The heat shrinkage ratio is calculated from areas before and after shrinkage according to following formula:

$$\text{Area shrinkage ratio (\%)} = (a-b)/a \times 100$$

Table 1

| | | | Example 1 | Example 2 | Comparative Example 1 | Comparative Example 2 |
|---|---|---|---|---|---|---|
| Thickness | | mm | 1.80 | 1.80 | 1.77 | 1.81 |
| Basis weight | | g/m$^2$ | 43 | 40 | 38 | 35 |
| Conveying speed ratio (step f/step e) | | - | 0.90 | 0.95 | 1.00 | 1.00 |
| Average fiber angle $\theta$ | $\theta_M$ in lengthwise cross-section | ° | 66.5 | 68.6 | 73.5 | 76.5 |
| | $\theta_C$ in widthwise cross-section | ° | 55.1 | 63.5 | 69.8 | 73.4 |
| Artificial urine permeation rate | | sec | 1.79 | 2.07 | 3.24 | 4.31 |

It is seen from the evaluation results on the artificial urine permeation rate of Example 1 and Comparative Example 2 in Table 1 that the artificial urine permeation rate is increased by decreasing the average fiber angle. This is because the fiber orientation is directed to the thickness direction TD by the decrease in the average fiber angle and the resistance encountered by artificial urine is lowered, facilitating permeation of artificial urine through the prepared sample.

It is seen from the evaluation results on the artificial urine permeation rate of Examples 1 and 2 and Comparative Example 1 in Table 1 that by reducing the ratio of the conveying speed in the step f to the conveying speed in the step e, the shrinking of the web in the length direction MD due to crimping of the latent crimping fiber in the second layer is easily obtained and therefore, the average fiber angle can be decreased. In the sample having a smaller average fiber angle, the fiber orientation can be more directed in the thickness direction TD and the resistance encountered by artificial urine can be reduced, so that the artificial urine permeation rate can be increased.

INDUSTRIAL APPLICABILITY

The nonwoven fabric sheet of the present invention can be suitably used as a surface sheet of an absorbent article such as a disposable diaper or sanitary napkin.

DESCRIPTION OF NUMERICAL REFERENCES

| | |
|---|---|
| 1 | Nonwoven fabric sheet |
| 2 | Ridge |
| 3 | Groove |
| 4 | First layer |
| 5 | Second layer |
| 11 | Vessel |
| 12 | Carding machine |
| 13 | Web |
| 14 | Vessel |
| 15 | Carding machine |
| 16 | Web |
| 17 | Endless belt |
| 18 | Stacked web |
| 19 | Suction drum |
| 20 | Nozzle |
| 21, 22, 23 | Nozzle lines |
| 24 | Web |

| 25 | Endless belt |
|---|---|
| 26 | First heat treatment drier |
| 27 | Endless belt |
| 28 | Second heat treatment drier |
| 29 | Endless belt |
| 30 | Endless belt |
| 31 | Roll |
| 40 | Floating drier |
| 41 | Mesh conveyer |
| 42 | Mesh conveyer |
| 43 | Hot air blowing port |
| A | Top surface |
| B | Bottom surface |

**Claims**

1. A nonwoven fabric sheet having length, width and thickness directions crossing each other at right angles and having a top surface and a bottom surface in the thickness direction, the top surface being subjected to formation of a plurality of ridges extending parallel in the length direction and a plurality of grooves extending in the length direction between adjacent ridges, wherein
the nonwoven fabric sheet comprises two layers of a first layer on the top surface side and a second layer on the bottom surface side, the first layer comprises a heat-fusible fiber, the second layer comprises a crimped fiber, and an average fiber angle in the cross-section parallel to the length direction is not more than 70°, wherein acute intersection angles including 90° and obtuse intersection angles larger than 90° are formed by the intersection of a fiber constituting the first layer, which appears in the cross-section parallel to the length direction passing through the apex of the ridge when the nonwoven fabric sheet is placed on a horizontal plane, with a line perpendicular to the horizontal plane, and the average fiber angle in the cross-section parallel to the length direction is an average value of the acute intersection angles.

2. The nonwoven fabric sheet as claimed in claim 1, wherein an average fiber angle in the cross-section parallel to the width direction is not more than 65°, wherein acute intersection angles including 90° and obtuse intersection angles larger than 90° are formed by the intersection of a fiber constituting the first layer, which appears in the cross-section parallel to the width direction when the nonwoven fabric sheet is placed on a horizontal plane, with a line perpendicular to the horizontal plane passing through the apex of the ridge, and the average fiber angle in the cross-section parallel to the width direction is an average value of acute intersection angles.

3. The nonwoven fabric sheet as claimed in claim 1 or 2, wherein the crimped fiber is a coiled three-dimensional crimped fiber.

4. The nonwoven fabric sheet as claimed in any one of claims 1 to 3, wherein the thickness of the ridge is from 0.3 to 5 mm, the difference in height between the apex of the ridge and the bottom of the groove is from 0.1 to 3 mm, the width of the ridge is from 1 to 10 mm, and the width of the groove is from 0.5 to 7 mm.

5. The nonwoven fabric sheet as claimed in any one of claims 1 to 4, wherein the basis weight of the first layer is from 10 to 100 $g/m^2$ and the basis weight of the second layer 5 is from 5 to 70 $g/m^2$.

6. The nonwoven fabric sheet as claimed in any one of claims 1 to 5, wherein the crimped fiber is contained in a large proportion in the ridge as compared with the groove.

7. A method for producing a nonwoven fabric sheet comprising:

    a) a step of passing a fiber aggregate comprising a latent crimping fiber through a carding machine and thereby opening out the fibers to form a web comprising a latent crimping fiber,
    b) a step of passing a fiber aggregate comprising a heat-fusible fiber through a carding machine and thereby opening out the fibers to form a web comprising a heat-fusible fiber,
    c) a step of laminating together the web comprising a latent crimping fiber and the web comprising a heat-fusible fiber to form a stacked web,

d) a step of spraying a fluid on the heat-fusible fiber-comprising web-side surface of the stacked web from a plurality of nozzles arranged in the width direction, while conveying the stacked web, to form a plurality of ridges extending parallel in the length direction and a plurality of grooves extending in the length direction between adjacent ridges on the stacked web,

f) a step of heating the web having formed thereon ridges and grooves, at a temperature lower than the fusion temperature of the heat-fusible fiber but high enough for the latent crimping fiber to develop crimp, by using means for reducing the resistance to crimp development of the latent crimping fiber, and

g) a step of heating the web in which the latent crimping fiber is crimped, at a temperature not lower than the fusion temperature of the heat-fusible fiber and thereby fusion-bonding the heat-fusible fibers to each other at the site of the fibers intersecting one another.

8.  The method as claimed in claim 7, wherein the means for reducing the resistance against crimp development of the latent crimping fiber is a method of conveying the web at a low conveying speed compared with the last step.

9.  The method as claimed in claim 7 or 8, wherein the means for reducing the resistance to crimp development of the latent crimping fiber is a floating drier.

10. The method as claimed in any one of claims 7 to 9, comprising, between the step d and the step f,

    e) a step of conveying the web having formed thereon ridges and grooves to the step f.

11. The method as claimed in any one of claims 7 to 10, comprising, after the step g,

    h) a step of cooling the web in which the heat-fusible fibers are fusion-bonded to each other.

12. An absorbent article comprising, as a surface sheet, the nonwoven fabric sheet claimed in any one of claims 1 to 6.

# Fig. 1

# Fig. 2

Fig.3

# Fig.4

(a)

P

20   20

(b)

21
22
23

20   20

P

Q

MD

CD

# Fig.5

21   22   23
18            24

17            25

19

# Fig.6

# Fig.7

LENGTH DIRECTION

WIDTH DIRECTION

(a)

LENGTH
DIRECTION

WIDTH DIRECTION

(b)

EP 2 537 969 A1

Fig.8

(b)

(a)

Fig.9

（a）

（b）

# Fig.10

FIBER ANGLE (°)

| | |
|---|---|
| 1 | 85 |
| 2 | 79 |
| 3 | 55 |
| 4 | 87 |
| 5 | 49 |
| 6 | 80 |
| 7 | 87 |
| 8 | 78 |
| 9 | 78 |
| 10 | 84 |
| 11 | 77 |
| 12 | 64 |
| 13 | 67 |
| 14 | 65 |
| 15 | 62 |
| 16 | 65 |
| 17 | 68 |
| 18 | 70 |
| 19 | 63 |
| 20 | 64 |
| 21 | 47 |
| 22 | 74 |
| 23 | 85 |
| 24 | 65 |
| 25 | 73 |
| 26 | 75 |
| 27 | 78 |
| 28 | 80 |
| 29 | 73 |
| 30 | 54 |
| 31 | 23 |
| 32 | 60 |
| 33 | 62 |
| 34 | 42 |
| 35 | 85 |
| 36 | 83 |
| 37 | 64 |
| 38 | 74 |
| 39 | 82 |
| 40 | 80 |
| 41 | 75 |
| 42 | 84 |
| 43 | 88 |
| 44 | 79 |
| 45 | 79 |
| 46 | 79 |
| 47 | 58 |
| 48 | 68 |
| AVERAGE | 70.8 |

<table>
<tr><td colspan="2"><strong>INTERNATIONAL SEARCH REPORT</strong></td><td>International application No.<br>PCT/JP2011/054208</td></tr>
</table>

**A. CLASSIFICATION OF SUBJECT MATTER**
*D04H1/54*(2006.01)i, *A61F13/511*(2006.01)i

According to International Patent Classification (IPC) or to both national classification and IPC

**B. FIELDS SEARCHED**

Minimum documentation searched (classification system followed by classification symbols)
D04H1/00-18/00, A61F13/15-13/84

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2009-185408 A (Kao Corp.),<br>20 August 2009 (20.08.2009),<br>claims 1, 4, 5; drawings<br>(Family: none) | 1-12 |
| A | WO 2009/001590 A1 (Uni-Charm Corp.),<br>31 December 2008 (31.12.2008),<br>claims; drawings<br>& EP 2161361 A1<br>claims; figures<br>& JP 2009-30218 A          & US 2010/0191207 A<br>& AU 2008268795 A          & EA 201000054 A<br>& CN 101790604 A          & KR 10-2010-0048998 A | 1-12 |

☐ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>04 April, 2011 (04.04.11) | Date of mailing of the international search report<br>12 April, 2011 (12.04.11) |
|---|---|
| Name and mailing address of the ISA/<br>Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2009030218 A